# FASCICULE DE BREVET EUROPEEN

(11) **EP 4 076 754 B1**
(45) Date de publication et mention de la délivrance du brevet: **20.03.2024**
(21) Numéro de dépôt: 20848858.5
(22) Date de dépôt: 18.12.2020
(51) Int. Cl.: B01L 3/00, C12M 1/12, F16J 13/12, F16J 15/06, G21F 5/12, B01L 1/02, B01J 3/03

(54) **CONTENEUR ÉTANCHE POURVU D'UNE BRIDE BI-MATIÈRE**
VERSIEGELTER BEHÄLTER MIT EINEM ZWEISTOFFFLANSCH
SEALED CONTAINER PROVIDED WITH A BI-MATERIAL FLANGE

(30) Priorité: 20.12.2019 FR 1915264
(43) Date de publication de la demande: 26.10.2022
(73) Titulaire: ABC Transfer, 37000 Tours (FR)
(72) Inventeur: FELIX, Julien, 41100 VENDOME (FR); SCHNEIDER, Jean-Luc, 41100 SAINT FIRMIN DES PRES (FR); GIRARD, Thierry, 75116 PARIS (FR)
(74) Mandataire: IP Trust
(86) Numéro de dépôt international: PCT/FR2020/052562
(87) Numéro de publication internationale: WO 2021/123687

(56) Documents cités:
- EP-A1- 2 091 051
- WO-A1-2013/110745
- DE-A1- 2 208 988
- FR-A1- 2 787 235
- FR-A1- 2 998 328

## Description

### DOMAINE TECHNIQUE DE L'INVENTION

L'invention concerne le domaine des conteneurs destinés à être raccordés de manière étanche à des enceintes stériles afin de permettre la mise en communication de leurs volumes respectifs sans contact avec ledit milieu extérieur.

L'invention concerne plus particulièrement un conteneur étanche comprenant un corps de conteneur présentant une ouverture de passage délimitée par une bride et obturée par une porte amovible montée sur la bride, un joint d'étanchéité annulaire étant situé entre la bride et la porte.

Le conteneur étanche selon l'invention est destiné notamment, mais non exclusivement au transfert de produits dangereux comme certains produits pharmaceutiques, biotechnologiques, biologiques, chimiques ou radioactifs, du transfert de composants tels que bouchons, flacons, pistons, seringues, etc., au transfert de dispositifs de contrôle environnementaux tels que des supports de milieu de culture, des compteurs particulaires, etc., du transfert de systèmes de nettoyage, du transfert de liquides, de poudres, d'outils, du transfert de déchets vers l'extérieur de l'enceinte et/ou du transfert de tout élément nécessaire à la production ou à la maintenance de la ligne de production.

### ÉTAT DE LA TECHNIQUE

De manière connue, le raccordement d'un conteneur étanche à une enceinte stérile pour le transfert de produits, sans rupture de la connexion et de l'étanchéité, est réalisé à l'aide de deux dispositifs de connexion, l'un équipant le conteneur, l'autre équipant l'enceinte. Chaque dispositif de connexion comporte une bride délimitant respectivement l'ouverture de passage dans le volume intérieur du conteneur ou de l'enceinte, chacune des ouvertures de passage étant obturées par une porte. La bride et la porte du conteneur sont aptes à être raccordées respectivement à la bride et à la porte de l'enceinte par une liaison à baïonnettes l'une de l'autre sous l'action d'un mouvement de rotation de la bride et porte associée du conteneur par rapport à la bride et porte de l'enceinte sur lesquelles elles sont accolées. Ainsi, les brides et les portes respectivement du conteneur et de l'enceinte sont pourvues respectivement d'encoches et d'oreilles internes ou externes.

Lors du mouvement de rotation, la porte du conteneur est désolidarisée de la bride associée.

Usuellement, les brides des conteneurs et des enceintes sont en inox. De telles brides présentent cependant l'inconvénient d'être sujettes aux effets de grippage. Lors de leur raccordement, les brides en inox subissent en effet des frottements importants causés par la rugosité de leur surface en contact ce qui peut conduire, sur la durée, à l'arrachement de matière au niveau de ces surfaces et ainsi entrainer le grippage de celles-ci.

Afin de pallier à ce problème de grippage inox/inox lors du raccordement des brides entre elles, des galets et des patins sont insérés dans les oreilles de chacune des brides. Ce type de conteneurs rencontre cependant plusieurs problématiques.

L'une des problématiques rencontrées avec ce type de conteneurs est le blocage et l'usure des galets provoqués par les frottements, l'autoclave et le lavage des conteneurs. Il est donc nécessaire de procéder à des maintenances régulières de ces galets.

Une autre problématique rencontrée est le démontage des galets. Avec le temps, les axes de galets par lesquels ils sont fixés, peuvent gripper et donc empêcher leur démontage, leur taille relative réduite ne permettant pas d'utiliser un outil d'extraction approprié.

Une autre problématique rencontrée est une mauvaise manipulation des conteneurs qui peut, dans certains cas, entrainer des dommages irréversibles de ces derniers. Outre les déformations du corps de conteneur, les déformations des oreilles externes des brides peuvent en particulier conduire à rendre le conteneur inutilisable, ce dernier ne pouvant plus être raccordé à une enceinte.

Afin de tenter de pallier ces problématiques, il a été proposé dans la demande WO2013/110745 un conteneur dont la bride est pourvue d'oreilles amovibles réalisées en une matière antifriction, comme par exemple une matière plastique. Un des inconvénients d'un tel arrangement est d'imposer une maintenance fastidieuse et peu aisée (nombreuses pièces à démonter).

L'invention vise à remédier à ces problèmes en proposant un conteneur étanche réduisant les risques de grippage lors de son raccordement à une enceinte tout en offrant une maintenance facilitée et ainsi une durée de vie supérieure à celles des conteneurs de l'art antérieur.

L'invention a également pour objet de proposer un conteneur étanche présentant un dispositif de connexion compact, dépourvu de toute zone de rétention d'eau.

L'invention a également pour objet de proposer un conteneur étanche robuste et durable dans son utilisation, mais aussi résistant dans la durée à des stérilisations par autoclave et des lavages répétés ainsi que d'offrir une nettoyabilité améliorée.

### OBJET DE L'INVENTION

L'invention est définie par les revendications jointes. À cet effet, l'invention propose un conteneur étanche comprenant un corps de conteneur présentant une ouverture de passage délimitée par une bride et obturée par une porte amovible montée sur la bride, un joint d'étanchéité annulaire situé entre la bride et la porte, la bride, la porte et le joint d'étanchéité formant un dispositif de connexion étanche raccordable à un dispositif de connexion complémentaire d'une enceinte stérile de façon à permettre une communication stérile entre le conteneur étanche et l'enceinte stérile, le conteneur étant remarquable en ce que la bride est constituée de deux manchons coaxiaux distincts, l'un des manchons définissant un corps de bride dit extérieur comprenant des moyens de raccordement pour permettre un raccordement du corps de conteneur à l'enceinte complémentaire, l'autre manchon, définissant un corps de bride dit intérieur pour permettre un raccordement de la porte sur la bride, le corps de bride extérieur étant réalisé en tout ou partie dans un matériau plastique résistant choisi parmi les plastiques résistant à des cycles de stérilisation à l'autoclave tandis que le corps de bride intérieur est réalisé en alliage métallique.

Ainsi, une bride bi-matière comprenant une partie en plastique choisie parmi les plastiques résistant à des cycles de stérilisation à l'autoclave associé et une partie en alliage métallique permet de s'affranchir de la problématique de grippage rencontrée avec les conteneurs de l'état de l'art. Cela permet également d'assurer une stérilisation du conteneur à des températures d'autoclave (de l'ordre de 135°C) sans augmentation du temps de traitement. Cela permet également d'offrir une maintenance simple et rapide du dispositif de connexion. Cela permet également d'améliorer la nettoyabilité du conteneur, les lumières nécessaires pour les galets dans les conteneurs de l'état n'étant plus requises. Selon l'invention, la bride est constituée uniquement de deux pièces, les moyens de raccordement du corps de conteneur à l'enceinte et les moyens de raccordement de la porte à la bride étant formés d'un seul tenant avec respectivement l'une de ces pièces. Ainsi, les moyens de raccordement du corps de conteneur à l'enceinte sont formés d'un seul tenant avec le corps de bride extérieur tandis que les moyens de raccordement de la porte sur la bride étant formés d'un seul tenant avec le corps de bride intérieur. Le corps de bride extérieur est assemblé sur le corps de bride intérieur de manière amovible (assemblage réversible). Cela permet ainsi de démonter facilement et de manière réversible le corps de bride extérieur qui correspond à la partie de la bride la plus exposée aux erreurs de manipulation. Le corps de bride extérieur est ainsi interchangeable en cas d'altération. Il devient ainsi une pièce de rechange, de sorte que les conteneurs formés ainsi d'une bride bi matière dont la partie extérieure est en plastique présentent une durée de vie allongée au regard de celle des conteneurs à brides réalisées en inox.

Avantageusement, le corps de bride extérieur est assemblé au corps de bride intérieur par des vis d'assemblage montées sur la face avant du conteneur.

Avantageusement, le corps de bride extérieur et le corps de bride intérieur délimitent une gorge radiale recevant un talon discal du joint d'étanchéité.

Avantageusement, le corps de bride intérieur présente une face interne aménagée pour former une rampe d'écoulement de liquide vers l'intérieur du corps du conteneur.

Avantageusement, le corps de bride extérieur est en PEEK (polyétheréthercétone).

Avantageusement, le corps de bride intérieur est formé d'un seul tenant avec le corps de conteneur.

Avantageusement, le corps de bride intérieur est en inox.

### BREVE DESCRIPTION DES FIGURES

D'autres caractéristiques et avantages de l'invention ressortiront de la description détaillée de l'invention qui va suivre en référence aux figures annexées et dans lesquelles :
- La figure 1 représente une vue schématique en coupe d'un conteneur selon l'invention ;
- La figure 2 représente une vue de dessus du conteneur de la figure 1 ;
- La figure 3 représente une vue de détail du conteneur de la figure 1 ;
- La figure 4 représente une vue éclatée du conteneur de la figure 1 ;
- La figure 5 représente une vue de face en perspective latérale du conteneur de la figure 1, la porte étant montrée retirée du conteneur ;
- La figure 6 représente une vue en coupe selon l'axe VI-VI du conteneur de la figure 5.

### DESCRIPTION DETAILLEE DE L'INVENTION

Dans ce qui suit, les termes « extérieur », « externe », « intérieur » et « interne » sont définis par rapport au conteneur. Ainsi, on désigne par « extérieur » et « externe » ce qui est situé hors de l'espace intérieur du conteneur, le plus éloigné de l'espace intérieur ou dirigé à l'opposé de l'intérieur de l'enceinte et par « intérieur » et « interne » ce qui est situé dans l'espace intérieur, le plus proche de l'espace intérieur ou dirigé vers l'intérieur du conteneur.

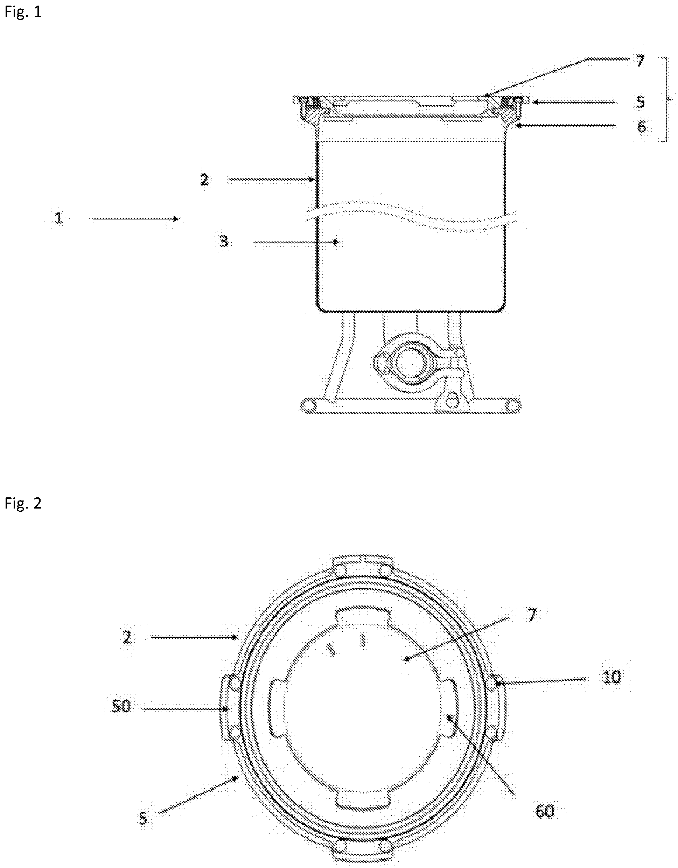

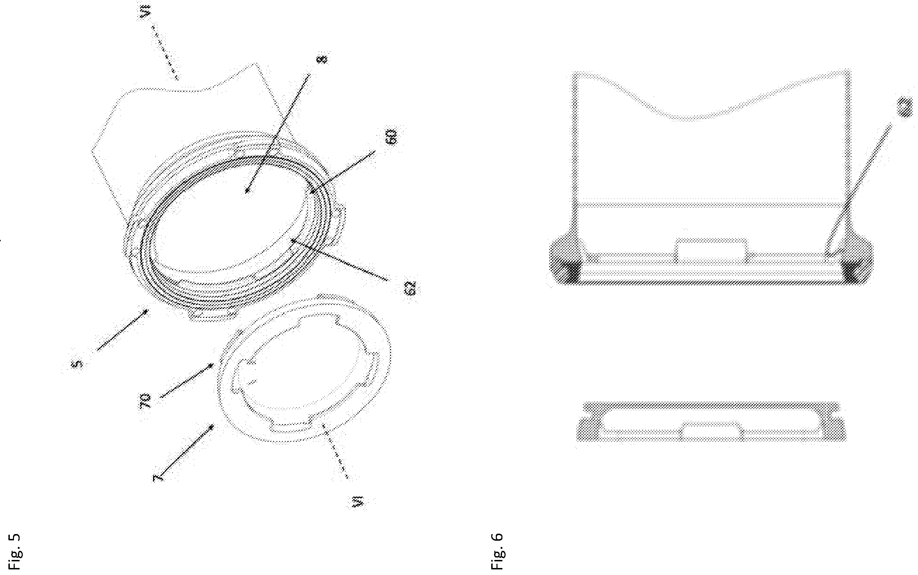

En relation avec les figures 1 à 6, il est décrit un conteneur 1 comprenant un corps de conteneur 2 rigide, avantageusement en inox, arrangé pour définir un volume intérieur 3 et une ouverture de passage 8.

Le conteneur 1 comporte également un dispositif de connexion 4 étanche apte à coopérer avec un dispositif de connexion complémentaire équipant une enceinte. Les dispositifs de connexion sont arrangés de manière à assurer une communication stérile entre le volume intérieur 3 dudit conteneur 1 et celui de l'enceinte lorsque le conteneur est raccordé à l'enceinte.

Le dispositif de connexion 4 comprend une bride délimitant l'ouverture de passage 8 et une porte 7 amovible fixée sur la bride de façon à obturer l'ouverture de passage 8. Selon l'invention, la bride est constituée de deux manchons 5, 6 distincts et indépendants l'un de l'autre. Ces manchons 5, 6 sont coaxiaux, le manchon 6 étant avantageusement engagé dans le manchon 5. Le manchon 5 définit un corps de bride dit extérieur 5 qui est arrangé pour permettre une connexion du corps de conteneur 2 à l'enceinte. L'autre manchon 6 définit quant à lui un corps de bride dit intérieur 6 arrangé pour permettre une connexion de la porte 7 sur la bride. On désigne par « corps de bride extérieur », le corps de bride le plus éloigné de l'espace intérieur du conteneur. Par analogie, on désigne par « corps de bride intérieur », le corps de bride le plus proche de l'espace intérieur du conteneur. Dans l'exemple illustré, le manchon 6 est engagé partiellement dans le manchon 5. Il s'agit d'un exemple d'arrangement, étant entendu qu'il peut être prévu un arrangement dans lequel les manchons 5, 6 sont disposés concentriquement l'un par rapport à l'autre.

Le dispositif de connexion 4 comporte des moyens de raccordement, de type à baïonnette, pour le raccordement du conteneur 1 à une enceinte. Ainsi, la bride 5, 6 et la porte 7 du conteneur comportent respectivement un arrangement d'oreilles externes et internes 50 et 70, et d'encoches 51 et 71, complémentaire à un arrangement d'oreilles et d'encoches équipant l'enceinte et formant un dispositif de connexion complémentaire.

Dans l'exemple illustré, la porte 7 est assemblée à la bride également par des moyens de raccordement de type à baïonnette. Ainsi, la porte 7 comporte un arrangement d'oreilles externe 72 et d'encoches 73 coopérant avec un arrangement d'oreilles internes 60 et d'encoche 61 portées par la bride.

Comme illustré sur les figures, le corps de bride extérieur 5 porte les oreilles externes 50 du système de baïonnette tandis que le corps de bride intérieur 6 porte les oreille internes 60 radiales permettant la retenue de la porte 7 sur la bride. Les oreilles externes 50 et les oreilles internes 60 sont formées d'un seul tenant avec les corps de bride les portant respectivement.

Afin de limiter les frottements, le corps de bride extérieur 5 est réalisé en tout ou partie dans un matériau plastique. Le matériau plastique sera choisi parmi les plastiques pour résister à des cycles de stérilisation à l'autoclave. Avantageusement, le corps de bride extérieur 5 sera réalisé en PEEK.

Le corps de bride intérieur 6 est quant à lui réalisé en alliage métallique, en particulier dans le même matériau que le corps de conteneur 2. Avantageusement, il est réalisé en inox. Dans l'exemple illustré, le corps de bride intérieur 6 est formé d'un seul tenant avec le corps de conteneur 2.

Le corps de bride extérieur 5 est assemblé sur le corps de bride intérieur 6 de manière amovible. Dans le mode de réalisation illustré, le corps de bride extérieur 5 est assemblé au corps de bride intérieur 6 par des moyens de fixation du type, dans l'exemple illustré, vis d'assemblage 10, qui traversent les oreilles externes 50 du corps de bride extérieur 5. Le corps de bride extérieur 5 et intérieur 6 sont assemblés par la face avant du conteneur. Par face avant, on entend la face du conteneur destinée à être placée en regard de l'enceinte. Avantageusement, les vis d'assemblage 10 sont masquées par des caches 11 affleurant la face avant du corps de bride extérieur 5. Les caches 11 sont réalisés de préférence en matière plastique afin de faciliter le frottement, et de préférence dans le même matériau que celui du corps de bride extérieur. Dans l'exemple décrit, les caches sont en PEEK.

Le conteneur 1 comporte en outre un joint d'étanchéité 13 annulaire porté par la bride. Le joint d'étanchéité 13 est couplé directement à la bride 5, 6. Ledit joint d'étanchéité 13 présente un corps de joint annulaire, de section avantageusement parallélépipédique, prolongé sur sa périphérie externe par un talon 14 discal enserré dans une gorge délimitée par le corps de bride extérieur 5 et le corps de bride intérieur 6. Le corps de joint annulaire présente avantageusement une face avant d'étanchéité 13A en contact avec la face arrière du corps de joint extérieur 16 et une face latérale d'étanchéité 13B destinée à être au contact de la porte 7 lorsque celle-ci est en position d'obturation de l'ouverture de passage 8. Ainsi, lorsque la porte 7 est en position d'obturation, le joint annulaire 13 est situé dans l'espace délimité par le corps de bride externe 5, le corps de bride interne 6 et la porte 7. Avantageusement, les faces d'étanchéité 13A et 13B sont non lisses de sorte à favoriser le frottement lors du raccordement du conteneur à l'enceinte, améliorant ainsi les efforts de connexion. Cela permet également de limiter la friction entre la surface du joint et la surface de contact de la bride ou de la porte suivant le cas. Les faces d'étanchéité peuvent ainsi être pourvues de stries, de crénelages, de dents, de picots ou présenter un état de surface favorisant les frottements. Le joint d'étanchéité 13 est réalisé en un matériau silicone ou autres matériaux minimisant les phénomènes de rémanence. Il peut être prévu également que le joint d'étanchéité soit réalisé en un matériau bactériostatique.

La forme du joint d'étanchéité 13 et son arrangement avec la porte et la bride permet de créer une zone d'étanchéité « pleine », c'est-à-dire sans cavité ou vide (désigné sous les termes de zones cachées) contrairement aux joints de l'art antérieur qui comportent de telles zones lesquelles favorisent la rétention de liquides de lavage et de bactéries.

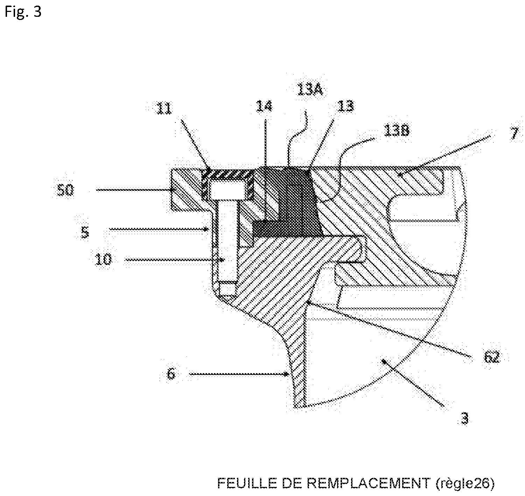

Afin d'améliorer l'écoulement du liquide de lavage, le corps de bride intérieur 6 présente avantageusement une face interne 62 aménagée pour former une rampe d'écoulement de liquide vers l'intérieur du corps du conteneur 1 comme illustré sur les figures 3, 5 et 6.

Dans ce qui précède, le corps de conteneur 2 est rigide. Il est bien entendu évident que le conteneur 1 selon l'invention ne se limite pas à ce type de conteneur 1 et qu'il peut être prévu par exemple un corps de conteneur 2 se présentant sous la forme d'une poche souple. Dans ce cas, la poche souple sera avantageusement maintenue en prise entre le corps de bride extérieur 5 et le corps de bride intérieur 6.

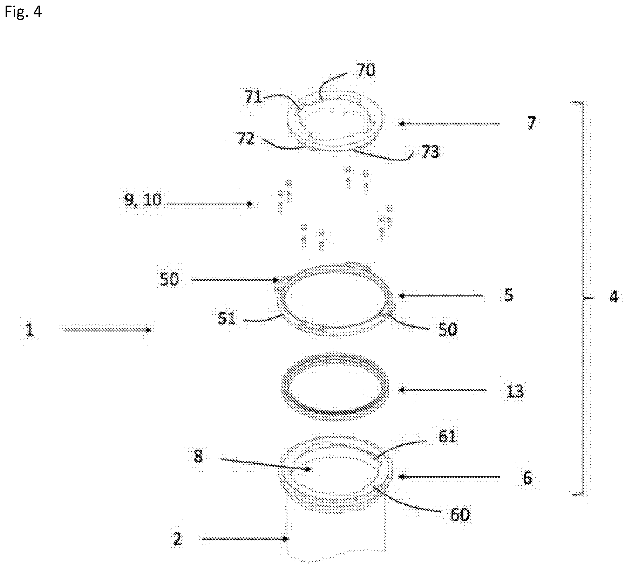

Du fait de l'arrangement des éléments le composant, le dispositif de connexion est assemblé par simple clipsage des éléments selon l'empilement illustré sur la figure 4.

L'invention est décrite dans ce qui précède à titre d'exemple. Il est entendu que la personne du métier est à même de réaliser différentes variantes de réalisation de l'invention sans pour autant sortir du cadre de l'invention qui est défini par les revendications jointes.

## Revendications

1. Conteneur (1) étanche comprenant :
- un corps de conteneur (2) définissant un espace intérieur et présentant une ouverture de passage (8) délimitée par une bride et obturée par une porte (7) amovible montée sur la bride et
- un joint d'étanchéité annulaire situé entre la bride et la porte (7),
- la bride, la porte (7) et le joint d'étanchéité formant un dispositif de connexion étanche raccordable à un dispositif de connexion complémentaire d'une enceinte stérile de façon à permettre une communication stérile entre le conteneur étanche et l'enceinte stérile,
**caractérisé en ce que** la bride est constituée de deux manchons coaxiaux distincts,
- l'un des manchons, définissant un corps de bride dit extérieur (5), comprenant des moyens de raccordement pour permettre un raccordement du corps de conteneur (2) à l'enceinte stérile, et
- l'autre manchon, définissant un corps de bride dit intérieur (6), comprenant des moyens de raccordement pour permettre un raccordement de la porte (7) sur la bride,
- le corps de bride extérieur (5) étant le plus éloigné de l'espace intérieur du conteneur tandis que le corps de bride intérieur (6) est le plus proche de l'espace intérieur du conteneur,
- le corps de bride extérieur (5) étant réalisé en tout ou partie dans un matériau plastique résistant choisi parmi les plastiques résistant à des cycles de stérilisation à l'autoclave tandis que le corps de bride intérieur (6) est réalisé en alliage métallique, le corps de bride extérieur (5) étant assemblé sur le corps de bride intérieur (6) de manière amovible.

2. Conteneur (1) étanche selon la revendication 1, **caractérisé en ce que** le corps de bride extérieur (5) est assemblé au corps de bride intérieur (6) par des vis d'assemblage montées sur la face avant du conteneur.

3. Conteneur (1) étanche selon la revendication 1 ou la revendication 2, **caractérisé en ce que** le corps de bride extérieur (5) et le corps de bride intérieur (6) délimitent une gorge radiale recevant un talon discal du joint d'étanchéité (13).

4. Conteneur (1) étanche selon l'une quelconque des revendications précédentes, **caractérisé en ce que** le corps de bride intérieur (6) présente une face interne aménagée pour former une rampe d'écoulement de liquide vers l'intérieur du corps du conteneur.

5. Conteneur (1) étanche selon l'une quelconque des revendications précédentes, **caractérisé en ce que** le corps de bride extérieur (5) est en PEEK.

6. Conteneur (1) étanche selon l'une quelconque des revendications précédentes, **caractérisé en ce que** le corps de bride intérieur (6) est formé d'un seul tenant avec le corps de conteneur (2).

7. Conteneur (1) étanche selon l'une quelconque des revendications précédentes, **caractérisé en ce que** le corps de bride intérieur (6) est en inox.

## Patentansprüche

1. Dichter Behälter (1), umfassend:
- einen Behälterkörper (2), der einen inneren Raum definiert und eine Durchgangsöffnung (8) vorweist, die durch einen Flansch begrenzt ist und durch eine abnehmbare Tür (7) verschlossen ist, die an dem Flansch befestigt ist, und
- eine ringförmige Dichtung, die sich zwischen dem Flansch und der Tür (7) befindet,
- wobei der Flansch, die Tür (7) und die Dichtung eine dichte Anschlussvorrichtung ausbilden, die mit einer komplementären Anschlussvorrichtung eines sterilen Gehäuses verbindbar ist, um eine sterile Kommunikation zwischen dem dichten Behälter und dem sterilen Gehäuse zu ermöglichen,
**dadurch gekennzeichnet, dass** der Flansch aus zwei verschiedenen koaxialen Hülsen besteht,
- wobei eine der Hülsen einen sogenannten äußeren Flanschkörper (5) definiert, umfassend Verbindungsmittel zum Ermöglichen einer Verbindung des Behälterkörpers (2) mit dem sterilen Gehäuse, und
- wobei die andere Hülse einen sogenannten inneren Flanschkörper (6) definiert, umfassend Verbindungsmittel zum Ermöglichen einer Verbindung der Tür (7) an dem Flansch,
- wobei der äußere Flanschkörper (5) von dem inneren Raum des Behälters am weitesten entfernt ist, wohingegen der innere Flanschkörper (6) dem inneren Raum des Behälters am nächsten liegt,
- wobei der äußere Flanschkörper (5) vollständig oder teilweise aus einem widerstandsfähigen Kunststoffmaterial hergestellt ist, das aus Kunststoffen ausgewählt ist, die gegenüber Sterilisationszyklen mit dem Autoklaven widerstandsfähig sind, wohingegen der innere Flanschkörper (6) aus einer Metalllegierung hergestellt ist, wobei der äußere Flanschkörper (5) auf dem inneren Flanschkörper (6) auf abnehmbare Weise montiert ist.

2. Dichter Behälter (1) nach Anspruch 1, **dadurch gekennzeichnet, dass** der äußere Flanschkörper (5) an dem inneren Flanschkörper (6) durch Montageschrauben montiert ist, die auf der vorderen Fläche des Behälters befestigt sind.

3. Dichter Behälter (1) nach Anspruch 1 oder 2,
**dadurch gekennzeichnet, dass** der äußere Flanschkörper (5) und der innere Flanschkörper (6) eine radiale Rille begrenzen, die einen Scheibenansatz der Dichtung (13) aufnimmt.

4. Dichter Behälter (1) nach einem der vorstehenden Ansprüche,
**dadurch gekennzeichnet, dass** der innere Flanschkörper (6) eine Innenfläche vorweist, die zum Ausbilden einer Rampe zum Strömen von Flüssigkeit in Richtung eines Inneren des Körpers des Behälters eingerichtet ist.

5. Dichter Behälter (1) nach einem der vorstehenden Ansprüche,
**dadurch gekennzeichnet, dass** der äußere Flanschkörper (5) aus PEEK besteht.

6. Dichter Behälter (1) nach einem der vorstehenden Ansprüche,
**dadurch gekennzeichnet, dass** der innere Flanschkörper (6) aus einem Stück mit dem Behälterkörper (2) ausgebildet ist.

7. Dichter Behälter (1) nach einem der vorstehenden Ansprüche,
**dadurch gekennzeichnet, dass** der innere Flanschkörper (6) aus Edelstahl besteht.

## Claims

1. Sealed container (1) comprising:
- a container body (2) defining an interior space and having a through opening (8) which is delimited by a flange and closed by a removable door (7) which is mounted on the flange, and
- an annular seal located between the flange and the door (7),
- the flange, the door (7) and the seal forming a sealed connection device which is connectable to a complementary connection device of a sterile enclosure so as to allow sterile communication between the sealed container and the sterile enclosure,
**characterized in that** the flange consists of two separate coaxial sleeves,
- one of the sleeves, defining a so-called outer flange body (5), comprising connection means to allow connection of the container body (2) to the sterile enclosure, and
- the other sleeve, defining a so-called inner flange body (6), comprising connection means to allow connection of the door (7) to the flange,
- the outer flange body (5) being furthest from the interior space of the container while the inner flange body (6) is closest to the interior space of the container,
- the outer flange body (5) being made entirely or partially of a resistant plastics material chosen from plastics resistant to autoclave sterilization cycles, while the inner flange body (6) is made of a metal alloy, the outer flange body (5) being removably assembled on the inner flange body (6).

2. Sealed container (1) according to claim 1, **characterized in that** the outer flange body (5) is assembled to the inner flange body (6) by assembly screws mounted on the front face of the container.

3. Sealed container (1) according to claim 1 or claim 2, **characterized in that** the outer flange body (5) and the inner flange body (6) delimit a radial groove receiving a disc heel of the seal (13).

4. Sealed container (1) according to any one of the preceding claims, **characterized in that** the inner flange body (6) has an internal face arranged to form a ramp for liquid to flow towards the interior of the container body.

5. Sealed container (1) according to any one of the preceding claims, **characterized in that** the outer flange body (5) is made of PEEK.

6. Sealed container (1) according to any one of the preceding claims, **characterized in that** the inner flange body (6) is formed in one piece with the container body (2).

7. Sealed container (1) according to any one of the preceding claims, **characterized in that** the inner flange body (6) is made of stainless steel.
